# EUROPÄISCHE PATENTSCHRIFT

(11) **EP 3 672 829 B1**
(45) Veröffentlichungstag und Bekanntmachung des Hinweises auf die Patenterteilung: **21.09.2022**
(21) Anmeldenummer: 19794956.3
(22) Anmeldetag: 23.10.2019
(51) Int. Cl.: A61B 5/01, A61B 5/16, A61B 5/18, B60N 2/00, B60N 5/00, A61B 5/00

(54) **VERFAHREN ZUM ÜBERWACHEN EINES FAHRERS EINES FAHRZEUGS MITTELS EINES MESSSYSTEMS**
METHOD FOR MONITORING A DRIVER OF A VEHICLE BY MEANS OF A MEASURING SYSTEM
PROCÉDÉ POUR SURVEILLER UN CONDUCTEUR D'UN VÉHICULE AU MOYEN D'UN SYSTÈME DE MESURE

(30) Priorität: 14.11.2018 DE 102018128550
(43) Veröffentlichungstag der Anmeldung: 01.07.2020
(73) Patentinhaber: B-Horizon GmbH, 93053 Regensburg (DE)
(72) Erfinder: KABANY, Mohammad, 93053 Regensburg (DE)
(74) Vertreter: Peters, Andreas
(86) Internationale Anmeldenummer: PCT/EP2019/078890
(87) Internationale Veröffentlichungsnummer: WO 2020/099083

(56) Entgegenhaltungen:
- DE-A1-102016 003 125
- DE-A1-102017 111 908
- US-B1- 6 392 550

## Beschreibung

Die vorliegende Erfindung betrifft ein Verfahren zum Überwachen eines Fahrers eines Fahrzeugs mittels eines Messsystems sowie eine entsprechende Vorrichtung gemäß den Ansprüchen 1 und 9.

Die DE 10 2016 003125 A1 bezieht sich auf ein Verfahren zur Bestimmung des Zustands eines Fahrzeuginsassen in einem Fahrzeug. Nach der Erfindung wird eine Bewegung des Fahrzeuginsassen mittels mindestens einer Erfassungseinheit detektiert, wobei Fahrzeugdaten durch Anwendungen des vom Fahrzeuginsassen während der Fahrt benutzten Fahrzeugs erfasst werden, die erfassten Daten zusammen mit den Fahrzeugdaten ausgewertet werden, und in Abhängigkeit von den ausgewerteten Daten ein aktueller Zustand des Fahrzeuginsassen ermittelt wird, wobei bei Erkennung einer Bewegungslosigkeit mindestens eine Warnung an die Fahrzeuginsassen ausgegeben wird, und in Abhängigkeit von der ermittelten Bewegungslosigkeit eine Anzahl verschiedener, durch die Fahrzeuginsassen aktivierbarer Anwendungen im Fahrzeug angezeigt wird.

Die DE 10 2017 111908 A1 zeigt einen Fahrzeugsitz, umfassend zumindest ein Betätigungsmittel, ausgelegt, um auf einen Teil des Körpers eines Insassen des Sitzes zu wirken, Dabei wird das Betätigungsmittel in Abhängigkeit von zumindest einer Messung eines Sensors für Schweiß des Insassen des Sitzes gesteuert.

Das hier unter anderem vorgeschlagene Verfahren umfasst den ersten Schritt, bei dem zumindest ein Messsystem zur Messung von Druck und/oder Feuchtigkeit bereitgestellt wird, wobei das Messsystem mit mindestens einem Fahrzeugelement gekoppelt oder mit mindestens einem integriert verbaut ist. Hierbei weist das Messsystem zumindest einen Sensor zur Messung eines, vorzugsweise nur eines, Stresslevels, des Fahrers auf, sodass durch den Sensor Druck und/oder Feuchtigkeit gemessen wird.

In einem zweiten Schritt werden anschließend die gemessenen Stresslevelwerte, also die Druck- und/oder Feuchtigkeitswerte, an eine Verarbeitungseinheit des Messsystems weitergeleitet.

In einem dritten Schritt wird ein Stressniveau des Fahrers basierend auf den Stresslevelwerten erfasst und erkannt.

In einem vierten Schritt wird eine ausgewählte Aktion basierend auf der Erkennung durch das Messsystem ausgewählt, wobei die Aktion aus einer Liste ausgewählt ist, bestehend aus: Einrichten eines Anrufs zu einer entfernten Unterstützungsstelle, Übertragen der Stresslevelwerte zu einer entfernten Unterstützungsstelle, Erzeugen eines akustischen Alarms und Erzeugen eines visuellen Alarms, Anpassung einer Lautstärke von Lautsprechern in dem Fahrzeug, Anpassen einer Sitzposition eines Fahrzeugsitzes des Fahrzeugs, sowie Anzeige von Pausenempfehlungen an den Fahrer.

Als mögliche Aktionen kommt jedoch auch eine Musikanpassung in Frage. Dies kann heißen, dass der Musikkanal gewechselt wird. Hierzu ist denkbar, dass der Musikkanal in einem Fahrzeugradio automatisch in eine Musik-App gewechselt wird. Hierzu ist vorstellbar, dass diese Musik-App in der Verarbeitungseinheit oder einer sonstigen Einheit vorab hinterlegt ist, und bei Überschreiten zumindest eine Stresslevelwertes diese Musik-App vollautomatisch geladen wird. Diese Musik-App kann dann vollautomatisch zumindest einen vorab hinterlegten Musiktitel abspielen, insbesondere um den Fahrer zu beruhigen, sodass die Stresslevelwerte sinken. Alternativ oder zusätzlich dazu kann auch eine interne Fahrzeugbeleuchtung eingestellt werden. Eine entsprechende Einstellung kann als "Ambient light" bezeichnet werden, sodass eine Lichtfarbe, Lichtintensität und eine Helligkeit eingestellt wird.

Insbesondere wird in der vorliegenden Erfindung unter anderem eine Druckmessung und/oder Feuchtigkeitsmessung in Zusammenhang mit einem Stresslevel beansprucht. Die Feuchtigkeitsmessung basiert insbesondere auf der Ausschüttung von Schweiß auf der Haut des Fahrers, welcher von außen und mit der Haut ein Nutzungsobjekt des Fahrzeugs (Lenkrad, Joystick, Radio, etc.) berührt.

Unter einem Stresslevel versteht man die Beanspruchung (Auswirkung der Belastungen) des Menschen durch innere und äußere Reize oder Belastungen (objektive, auf den Menschen einwirkende Faktoren sowie deren Größen und Zeiträume). Diese können sowohl künstlich als auch natürlich, sowohl biotisch als auch abiotisch sein, sowohl auf den Körper als auch die Psyche des Menschen einwirken und letztlich als positiv oder negativ empfunden werden oder sich auswirken. Die Bewältigung der Beanspruchung ist von den persönlichen (auch gesundheitlichen) Eigenschaften und kognitiven Fähigkeiten der individuellen Person abhängig, der Umgang mit einer Bedrohung wird auch Coping genannt. Einsetzbare Verhaltensweisen sind z. B. Aggression, Flucht, Verhaltensalternativen, Akzeptanz, Änderung der Bedingung oder Verleugnung der Situation.

Als "positiver Stress" bzw. Eustress (Die griechische Vorsilbe εù̃ (eu) bedeutet "wohl, gut, richtig, leicht") werden diejenigen Stressoren bezeichnet, die den Organismus zwar beanspruchen, sich aber positiv auswirken. Positiver Stress erhöht die Aufmerksamkeit und fördert die maximale Leistungsfähigkeit des Körpers, ohne ihm zu schaden. Eustress tritt beispielsweise auf, wenn ein Mensch zu bestimmten Leistungen motiviert ist, dann Zeit und Möglichkeiten hat, sich darauf vorzubereiten oder auch wenn eine (ggf. auch längere oder schwere) Krisensituation oder Krankheit dennoch positiv angegangen, bewältigt (Bewältigungsstrategie) und überwunden werden kann. Im Resultat können sogar Glücksmomente empfunden werden. Eustress wirkt sich auch bei häufigem, längerfristigem Auftreten positiv auf die psychische oder physische Funktionsfähigkeit eines Organismus aus.

Stress wird erst dann negativ empfunden, wenn er häufig oder dauerhaft auftritt und körperlich und/oder psychisch nicht kompensiert werden kann und deshalb als unangenehm, bedrohlich oder überfordernd gewertet wird. Insbesondere können negative Auswirkungen auftreten, wenn die individuelle Person (auch durch ihre Interpretation der Reize) keine Möglichkeit zur Bewältigung der Situation sieht oder hat. Beispiele dafür sind Klausuren ohne Zeit oder Fähigkeit zum Lernen, eine trotz Ärztebesuch unklare oder nicht anerkannte Erkrankung (vgl. Semmelweis-Reflex), eine durch Lärm unerträgliche Wohnung ohne Möglichkeit zum Umzug, o. ä. In diesem Fall kann dauerhaft negativer Stress (auch Disstress oder Dysstress, engl. distress; die griechische Vorsilbe δú 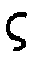 (dys) bedeutet "miss-, schlecht") gegebenenfalls durch geeignete Hilfen oder Stressbewältigungsstrategien verhindert werden. Abiotische Stressfaktoren wären z. B. physikalischer Natur, etwa Kälte, Hitze, Lärm, Abgase sowie natürliche und künstliche Strahlungen. Zu letzteren zählen etwa starke und übermäßig lange Sonneneinstrahlung oder sonstige, etwa hochfrequente oder radioaktive oder elektromagnetische Strahlungen. Weiterhin toxische Substanzen, z. B. Weichmacher, wie etwa Diethylhexylphthalat (DEHP) in PVC-Fußbodenbelägen oder Kinderspielzeug, (Zigaretten)Rauch und die darin enthaltenen Stoffe, Belastungen des Trinkwassers, übermäßiger und regelmäßiger Alkoholkonsum, vitalstoffarme Ernährung oder die zunehmend in einer Vielzahl von Produkten und Anwendungsverfahren der Landwirtschaft (z. B. "Krautregulierung" durch Glyphosate) angewandten - und dadurch in den menschlichen Körper aufgenommenen - Pestizide.

Biotische Faktoren wären beispielsweise Belastungen durch Krankheitserreger oder Tumore, auch chronische und autoimmune Entzündungsprozesse, die jedoch wiederum durch die oben genannten abiotischen Faktoren (Stressoren mit Auswirkungen auf Zell-Stoffwechsel und Immunsystem) beeinflusst sind. Auf emotionaler Ebene können auch psychische Belastungen wie Mobbing, bestimmte eigene Einstellungen und Erwartungshaltungen eines Menschen oder z. B. seiner Eltern, und weiterhin Befürchtungen, Stressoren sein (psychosoziale Stressfaktoren).

Stress ist also zunächst die Beanspruchung des Körpers durch solche Stressoren. Daraufhin erfolgt eine Reaktion und ggf. Anpassung des Körpers auf und an diese Faktoren, ggf. mit Hilfe von außen. Disstress führt zu einer stark erhöhten Anspannung des Körpers (Ausschüttung bestimmter Neurotransmitter und Hormone, z. B. Adrenalin und Noradrenalin) und auf Dauer zu einer Abnahme der Aufmerksamkeit und Leistungsfähigkeit. Stress bzw. Disstress wirkt erst dann schädigend auf den menschlichen Organismus, wenn Beanspruchung über den Bereich der nach seiner individuellen Physis und Psyche bzw. gesundheitlichen Verfassung möglichen Anpassung und Reparaturfunktionen (z. B. DNA-Reparatur) des individuellen Menschen, bzw. dessen Organismus, hinaus (chronischer Stress/Einwirkungsdauer, Übermaß, ggf. multiple Faktoren) erfolgt.

Gemäß zumindest einer Ausführungsform werden durch den Sensor nur der Druck und die Feuchtigkeit gemessen, um ein Stresslevel des Fahrers festzustellen.

Gemäß zumindest einer Ausführungsform ist der Sensor in einem Lenkrad und/oder einem Joystick und/oder einem Fahrzeugsitz eines Fahrzeugs verbaut, sodass der Fahrer das Lenkrad und/oder den Joystick und/oder den Fahrzeugsitz unmittelbar berührt.

Gemäß zumindest einer Ausführungsform sind in einem Speicher der CPU Grenzwerte von Druck und/oder Feuchtigkeit hinterlegt, wobei die jeweils zeitdiskret oder kontinuierlich gemessenen Druck- und Temperaturwerte mit den in dem Speicher der CPU hinterlegten Werten verglichen werden, wobei bei einer Überschreitung zumindest einer dieser Werte (Feuchtigkeit und Druck) von der CPU eine Aktion (zum Beispiel eine der obigen Aktionen) zur Ausführung bestimmt wird.

Gemäß zumindest einer Ausführungsform sind in einem Speicher der CPU Faktorgrenzwerte aus Druck und Feuchtigkeit hinterlegt, wobei die jeweils zeitdiskret oder kontinuierlich gemessenen Druck- und Temperaturwerte mit den in dem Speicher der CPU hinterlegten Werten verglichen werden, wobei bei einem Überschreiten zumindest einer dieser Werte (Feuchtigkeit und Druck) von der CPU eine Aktion (zum Beispiel eine der obigen Aktionen) zur Ausführung bestimmt wird, wobei der Faktorgrenzwert als ein Faktor des jeweiligen Druck- und Feuchtigkeitswerts definiert ist, insbesondere wobei beide Werte zum gleichen Zeitpunkt von dem Sensor gemessen werden.

Gemäß zumindest einer Ausführungsform werden innerhalb eines vorbestimmten Messzeitintervalls der Druck und die Feuchtigkeit jeweils zu verschiedenen Zeitpunkten gemessen. Es wurde die Erfahrung gemacht, dass bei einer Stressreaktion zunächst der Druck ansteigt und erst danach zeitverzögert eine Schweißbildung einsetzt. Einfach gesagt, umgreift der Fahrer zum Beispiel ein Lenkrad im Falle einer Stresssituation zunächst besonders stark, wobei erst danach eine entsprechende Schweißbildung einsetzt. Es können Wertepaare aus den Druck- und den dazugehörigen Feuchtewerte gebildet werden.

Erfindungsgemäß wird innerhalb des Messzeitintervalls zuerst der Druck und/oder die Temperatur und erst danach die Feuchtigkeit und/oder die Temperatur gemessen, wobei innerhalb jedes Messzeitintervalls nur einmal der Druck und nur einmal die Feuchtigkeit gemessen werden.

Gemäß zumindest einer Ausführungsform ist ein zeitlicher Abstand von zwei unmittelbar in zeitlicher Hinsicht benachbarten Messintervallen größer als der Zeitraum zumindest eines der Messintervalle, insbesondere wobei in dadurch generierten Messpausen keinerlei Messung stattfindet. Eine solche Messung kann insofern zeitgetaktet und nicht zeitkontinuierlich vorgenommen werden.

Des Weiteren betrifft die vorliegende Anmeldung eine Vorrichtung zum Überwachen eines Fahrers eines Fahrzeugs mittels eines Messsystems zur Durchführung des Verfahrens.

Gemäß zumindest einer Ausführung umfasst die Vorrichtung zumindest ein Messsystem zur Messung von Druck und/oder Feuchtigkeit, wobei das Messsystem, welches mit mindestens einem Fahrzeugelement gekoppelt oder mit mindestens einem integriert verbaut oder verbaubar ist, wobei das Messsystem zumindest einen Sensor zur Messung eines, vorzugsweise nur eines, Stresslevels, des Fahrers aufweist, sodass durch den Sensor Druck und/oder Feuchtigkeit messbar ist.

Die vorliegende Erfindung betrifft des Weiteren ein Messsystem zur Messung von Druck und/oder Feuchtigkeit sowie ein Verfahren zur Messung von Druck und/oder Feuchtigkeit. Das hier beschriebene Messsystem kann ein solches sein, welches in dem obig beschriebenen Verfahren zum Überwachen eines Fahrers eines Fahrzeugs mittels eines Messsystems sowie der entsprechenden Vorrichtung verwendet wird.

Das unter anderem erfindungsgemäße Messsystem zur Messung von Druck und/oder Feuchtigkeit umfasst zumindest einen Sensor zur Messung von Druck und/oder Feuchtigkeit, wobei der Sensor zumindest einen Kondensator mit zumindest zwei Elektroden umfasst, welche, insbesondere in einer horizontalen Richtung, entlang eines und auf einem, insbesondere flexiblen, Trägermaterial zueinander angeordnet sind, wobei zwischen den Elektroden zumindest eine dielektrische Schicht angeordnet ist.

Die horizontale Richtung ist vorzugsweise eine Haupterstreckungsrichtung des flexiblen Trägermaterials.

"Flexibel" heißt in diesem Zusammenhang, dass das Trägermaterial zumindest stellenweise biegsam und damit elastisch ist.

Insbesondere kann es sich bei dem Trägermaterial um einen Webstoff oder um einen sonstigen Bekleidungsstoff wie zum Beispiel ein Polyester handeln.

Die dielektrische Schicht beabstandet damit die beiden Elektroden in einer horizontalen und/oder in einer dazu senkrechten Querrichtung.

Erfindungsgemäß ist auf einer dem Trägermaterial abgewandten Seite zumindest eine Elektrode und/oder der dielektrischen Schicht zumindest stellenweise, zumindest eine, zumindest teilweise flüssigkeitsdurchlässige und/oder flüssigkeitsabsorbierende Feuchteschicht angeordnet, wobei somit die zumindest eine Elektrode und/oder dielektrische Schicht in einer Querrichtung zwischen dem Trägermaterial und der Feuchteschicht angeordnet sind, sodass eine Kapazität durch die auf die dielektrische Schicht zumindest teilweise treffende Flüssigkeit, zumindest teilweise verändert, wobei eine Verarbeitungseinheit dazu eingerichtet und dafür vorgesehen ist, die Messwerte des Sensors zu messen und/oder zu speichern, sodass ein kapazitiver Feuchtesensor entsteht.

Ein kapazitiver Feuchtesensor ist im Prinzip ein Kondensator, dessen Dielektrikum vorzugsweise aus einem hygroskopischen Polymerschicht besteht, die entsprechend der Feuchtigkeit der Umgebungsluft Feuchtigkeit aufnimmt (absorbiert) oder abgibt (desorbiert) bis ein Gleichgewichtszustand (Diffusionsgefälle ist = 0) erreicht ist. Dabei verändert sich die Dielektrizitätskonstante des Polymermaterials als Funktion eines Feuchtegehalts.

Die Aufgabe der Verarbeitungseinheit besteht unter anderem darin, vorzugsweise auch aus einer gemessenen Umgebungstemperatur und dem feuchtigkeitsabhängigen Kapazitätswert des Sensors die relative Feuchte möglichst genau zu ermitteln.

Erfindungsgemäß werden von der Verarbeitungseinheit die von dem Sensor gemessenen Daten an eine zentrale CPU (Central Processing Unit) gesendet, wobei diese Daten von der Verarbeitungseinheit verarbeitet werden. Die CPU und die Verarbeitungseinheit sind vorzugsweise verschieden voneinander. Die CPU und die Verarbeitungseinheit sind zum Beispiel voneinander beabstandet angeordnet. Insbesondere können die Verarbeitungseinheit und die CPU nicht auf einem gemeinsamen Träger und/oder Substrat angeordnet sein, sofern der Träger nicht das Trägermaterial, also zum Beispiel einem Textil, ist.

Auch ist denkbar, dass die hier beanspruchte Vorrichtung und insbesondere die Sensoren, auf einer Innenfläche eines Reifen verbaut sind. Auch ist denkbar, dass die Sensoren sogar mit in das Material des Reifen eingefügt sind. Dabei ist vorstellbar, dass die Sensoren alle in das Material eingefügt und damit von dem Material des Reifens ummantelt sind und die Verarbeitungseinheiten auf der Innenfläche des Reifens angeordnet sind. Alternativ können jedoch auch die Verarbeitungseinheiten in das Material des Reifens eingefügt sein. Die

Sensoren können dann den Reifeninnendruck, die Reifeninnentemperatur und/oder die Einzel- oder Gesamtlaufzeit des Reifens erfassen.

Gemäß zumindest einer Ausführungsform umfasst das Messsystem zumindest eine Vorrichtung zur Messung von Druck und/oder Feuchtigkeit, wobei die Vorrichtung zumindest einen Sensor zur Messung von Druck und/oder Feuchtigkeit aufweist, wobei der Sensor zumindest einen Kondensator mit zumindest zwei Elektroden umfasst, welche insbesondere in einer horizontalen Richtung entlang eines und auf einem insbesondere flexiblen Trägermaterial zueinander angeordnet sind, wobei zwischen den Elektroden zumindest eine dielektrische Schicht angeordnet ist.

Erfindungsgemäß ist auf eine dem Trägermaterial abgewandten Seite zumindest eine Elektrode und/oder dielektrische Schicht zumindest stellenweise, zumindest eine, zumindest teilweise flüssigkeitsdurchlässige und/oder flüssigkeitsabsorbierende Schicht (= Feuchteschicht) angeordnet, wobei somit die zumindest eine Elektrode und/oder dielektrische Schicht in Querrichtung zwischen dem Trägermaterial und der Feuchteschicht angeordnet sind, sodass eine Kapazität durch die auf die dielektrische Schicht zumindest teilweise treffende Flüssigkeit, zumindest teilweise verändert wird, wobei eine Verarbeitungseinheit dazu eingerichtet und dafür vorgesehen ist diese Messwerte des Sensors zu messen und/oder zu speichern, sodass ein kapazitiver Feuchtesensor entsteht.

Erfindungsgemäß werden von der Verarbeitungseinheit die von dem Sensor gemessenen Daten an eine zentrale CPU (Central Processing Unit) gesendet, wobei diese Daten von der Verarbeitungseinheit verarbeitet werden.

Der Sensor und/oder die Verarbeitungseinheit und/oder die zentrale CPU können mittels einer Batterie oder einer Festnetzstromversorgung mit elektrischer Energie versorgt werden.

Alternativ oder zusätzlich ist die Erzeugung von elektrischer Energie zur Versorgung des Sensors und/oder Verarbeitungseinheit mittels sogenannten "Energy Harvesting" möglich.

Als Energy Harvesting (wörtlich übersetzt *Energie-Ernten)* bezeichnet man die Gewinnung kleiner Mengen von elektrischer Energie aus Quellen wie Umgebungstemperatur, Vibrationen oder Luftströmungen für mobile Geräte mit geringer Leistung. Die dafür eingesetzten Strukturen werden auch als Nanogenerator bezeichnet. Energy Harvesting vermeidet bei Drahtlostechnologien Einschränkungen durch kabelgebundene Stromversorgung oder Batterien.

Möglichkeiten des Energy Harvesting:
- Piezoelektrische Kristalle erzeugen bei Krafteinwirkung, beispielsweise durch Druck oder Vibration, elektrische Spannungen. Diese Kristalle können an oder auf dem Trägermaterial angeordnet sein.
- Thermoelektrische Generatoren und pyroelektrische Kristalle gewinnen aus Temperaturunterschieden elektrische Energie. Diese Generatoren können an oder auf dem Trägermaterial angeordnet sein.
- Über Antennen kann die Energie von Radiowellen, eine Form von elektromagnetischer Strahlung, aufgefangen und energetisch verwendet werden. Ein Beispiel dafür sind die passiven RFIDs. Diese Antennen können an oder auf dem Trägermaterial angeordnet sein.
- Photovoltaik, elektrische Energie aus der Umgebungsbeleuchtung.
- Osmose.

Ein Energiespeicher der Vorrichtung kann Teil einer Verarbeitungseinheit sein. Hierzu können eine oder mehrere der Verarbeitungseinheiten einen solchen Energiespeicher (lokale Energiespeicher) aufweisen. Zum Beispiel weist nur eine oder einige der Verarbeitungseinheiten einen solchen Energiespeicher auf, sodass eine dieser Verarbeitungseinheiten eine andere Verarbeitungseinheit (nämlich eine solche, welche keinen Energiespeicher aufweist) mit elektrischer Energie versorgt.

Auch ist denkbar, dass die Energiespeichereinheit(en) der Verarbeitungseinheit(en) die CPU mit elektrischer Energie ganz oder teilweise versorgt. Zum Beispiel kann so die CPU an keine weiteren Energiespeicher und/oder Energieversorgungsleitungen angeschlossen sein.

Über das oben genannte Energy Harvesting kann zumindest einer der Energiespeicher geladen werden.

Die Energieübertragung zwischen den Sensoren und/oder den Verarbeitungseinheiten und/oder der CPU kann ganz oder teilweise drahtlos erfolgen.

Zu der drahtlosen Energieübertragungen im Nahfeld, auch als nicht strahlende Kopplung bezeichnet, zählt beispielsweise die indukvtive Kopplung, basierend auf dem magnetischen

Fluss. Häufig wird die Bezeichnung der drahtlosen Energieübertragung synonym für die induktive Energieübertragung verwendet, da diese in praktischen Anwendungen eine dominante Rolle einnimmt. Bei der nicht strahlenden Kopplung im Nahfeld spielen Wellenphänomene keine Rolle.

Zum Beispiel geschieht die drahtlose Energieübertragung zwischen den einzelnen Elementen mittels induktiver Kopplung, resonanter induktiver Kopplung und/oder kapazitiver Kopplung.

Gemäß zumindest einer Ausführungsform weist das Messsystem zumindest zwei Sensoren auf, wobei durch die Verarbeitungseinheit die Sensoren in Gruppen aus zumindest einem Sensor auf Basis zumindest einem der folgenden Kriterien unterteilt werden:
- Anordnungsort des Sensors oder der Sensoren auf dem Trägermaterial , wobei das Trägermaterial in Flächengebiete eingeteilt ist, und innerhalb eines Flächengebiets nur Sensoren einer Gruppe angeordnet sind,
- Flächenausdehnung eines Sensors.

Gemäß zumindest einer Ausführungsform umfasst das Messsystem zumindest zwei Vorrichtungen zur Messung von Druck und/oder Feuchtigkeit, wobei jede Verarbeitungseinheit Ihre von den Sensoren empfangenen Daten an die zentrale CPU weiterleitet. Die Datenverbindung zwischen der Verarbeitungseinheit und der zentralen CPU kann kabelgebunden (mit Datenverbindungen) oder aber Wireless erfolgen. Hierzu kann zumindest eine Verarbeitungseinheit eine Bluetooth Verbindung zu der zentralen CPU aufbauen.

Gemäß zumindest einer Ausführungsform umfasst zumindest eine Vorrichtung zumindest zwei Sensoren. Insofern kann eine Sensorgruppe bereits durch diese zwei Sensoren gebildet sein. Die beiden Sensoren könne dann durch eine gemeinsame Verarbeitungseinheit gesteuert und/oder geregelt werden.

Denkbar ist, dass die Vielzahl von Verarbeitungseinheiten ein Verarbeitungsnetzwerk ausbilden, wobei die Erfassung, Verarbeitung und/oder Weitergabe der Sensordaten und/oder der Verarbeitungsdaten eines jeden Sensors und/oder einer jeden Verarbeitungseinheit durch zumindest eine Steuerungseinrichtung (Master) gesteuert wird. Die Steuerungseinheit kann identisch mit der oben beschriebenen CPU sein.

Es ist jedoch auch möglich, dass eine oder mehrere der Verarbeitungseinheiten den Master darstellen, welcher die übrigen Verarbeitungseinheiten (Slave) und/oder die übrigen Sensoren (Slave) steuern.

Zum Beispiel kann eine der Verarbeitungseinheiten und/oder die CPU nach Inbetriebnahme der Vorrichtung (zum Beispiel nach einem Anschalten der Vorrichtung), solche Sensoren auswählen, welche für eine vorgebbare Nutzungsdauer in Betrieb genommen werden. Alternativ können auch alle oder einige Sensoren in Betrieb genommen werden, dann jedoch ist vorstellbar, dass eine Verarbeitungseinheit und/oder die CPU, insbesondere zum Zwecke der Energieersparnis, nur Daten einer vorgegebenen Anzahl (also weniger als alle Sensoren) von Sensoren an die CPU weiterleitet (Filterung).

Diese Master-Verarbeitungseinheit kann vorzugsweise als einzige Einheit mit der CPU kommunizieren.

Weiter alternativ oder zusätzlich ist vorstellbar, dass eine oder alle Verarbeitungseinheiten und/oder ein Sensor (Slave oder Master) direkt mit der CPU kommunizieren.

Gemäß zumindest einer Ausführungsform ist das Verarbeitungsnetzwerk mittels zumindest eines VLAN-Switches in zumindest zwei, nur logisch voneinander getrennte, Netzwerksegmente (VLANs) unterteilbar, wobei jedes der Erfassungselemente in Abhängigkeit von der Ansteuerung durch einen VLAN-Switch und/oder der Steuerungseinrichtung und somit durch jedes der Netzwerksegmente ansteuerbar ist.

Wird zum Beispiel eine sehr große Fläche (zum Beispiel ein Textil) mit einer Vielzahl von hier beanspruchten Sensoren und Verarbeitungseinheiten bestückt können in besonders einfacher Art und Weise dann einzelne Verarbeitungseinheiten und/oder Sensoren kategorisiert werden (nach verschiedenen Prioritäten etc.). Es wird somit in einer Ausführungsform statt einer physikalischen Netzwerkunterteilung eine "virtuelle", das heißt VLAN-Unterteilung gewählt. Dies gewährleistet nämlich, dass auf Veränderungen in der Kategorisierung der Verarbeitungseinheiten und/oder Sensoren besonders schnell und ohne aufwendige Umbauarbeiten reagiert werden kann.

Gemäß zumindest einer Ausführungsform umfasst das Messsystem zumindest ein Verarbeitungsnetzwerk, wobei mittels zumindest eines VLAN-Switches des Verarbeitungsnetzwerks, dieses in zumindest zwei, nur logisch voneinander getrennte, Netzwerksegmente (VLAN) unterteilbar ist, und wobei jeder Verarbeitungseinheiten und/oder jeder der Sensoren in Abhängigkeit von der Ansteuerung durch den VLAN-Switch durch jedes der Netzwerksegmente ansteuerbar sind.

Dazu kann der VLAN-Switch in zumindest einem der Verarbeitungseinheiten und/oder Sensoren oder in einem separaten Bauteil verbaut sein.

Gemäß zumindest einer Ausführungsform wird mittels des VLAN-Switches eine Priorisierung der einzelnen Netzwerksegmente insbesondere im Hinblick auf deren Datenaustausch durchgeführt.

Gemäß zumindest einer Ausführungsform ist jeder Verarbeitungseinheit und/oder jedem Netzwerksegment zumindest eine VLAN-ID zugeordnet, wobei über jede der VLAN-IDs zumindest ein Sensor oder eine andere Verarbeitungseinheit ansteuerbar ist. Einzelne Sensoren und/oder einzelne Verarbeitungseinheiten können ein eigenes Sub-Netzwerk bilden.

Um über Netzwerkgrenzen zu kommunizieren, werden im Stand der Technik statische projektdynamische Routen eingesetzt. Dieses Modell der Trennung ist klar und übersichtlich und wurde über Jahre angewandt. Dies hat jedoch die Nachteile, dass Broadcastanforderungen im Subnetz für alle Teilnehmer sichtbar sind und von den Endpunkten betrachtet werden müssten. Mit anderen Worten, konnten bisher verschiedene Endgeräte nur über entsprechende, jedem Subnetz zugeordnete, separate und physikalisch voneinander getrennte Switches angesteuert werden. Ein derartiger Aufbau ist jedoch besonders kostspielig und umfangreich im Design.

Wie obig bereits erwähnt, ist somit insbesondere auf die Ausgestaltung eines jeden Subnetzwerks mit einem separaten Switch und separaten physikalischen Datenleitungen verzichtet, sodass für das gesamte Netzwerk eine einzige physikalische Struktur angewandt werden kann, wobei diese physikalische Struktur, d. h. Netzwerkarchitektur, nur aufgrund einer logischen, insbesondere auch mathematischen Unterscheidung(d. h. gedacht), aufgetrennt wird.

Dabei bezeichnet die Abkürzung "VLAN-Switch" einen solchen Netzwerkswitch, welcher dazu eingerichtet und dafür vorgesehen ist, ein Netzwerk in Form eines Virtual Local Area Network (VLAN) mit zu betreiben.

Insofern wird somit durch die nunmehr beanspruchten Netzwerksegmente, welche jeweils in Form eines VLAN-Netzwerks ausgebildet sein können, ermöglicht, dass die Trennung des Netzwerks in mehrere logische Segmente, also die Netzwerksegmente, unterteilt wird.

Anders als bei der physikalischen Trennung durch die Zuordnung zu einem Switch Port werden bei der Trennung durch VLANs die Geräte durch eine VLAN-ID logisch getrennt. Dabei wird der Datenstrom jeder Station mit einer Kennung (dem VLAN-"Tag") versehen. Diese Kennung bestimmt die Zugehörigkeit eines Datenpakets zu einem bestimmten VLAN. Alle Geräte mit der gleichen VLAN-Kennung befinden sich nunmehr in einem logischen Netzwerk.

Insbesondere kann nämlich durch die logische Trennung der einzelnen Netzwerke ein Broadcast eingegrenzt werden. Broadcasts werden nämlich nur an Mitglieder des gleichen VLANS und nicht an alle am Switch hängenden Steuerelemente verteilt.

Dies trägt insofern auch nicht nur zu einer höheren Leistung, sondern auch zu mehr Sicherheit bei, denn der Datenverkehr wird auf weniger Adressaten eingeschränkt. Hinzu kommt, dass Benutzer oder die Steuerelemente in der Regel in einem VLAN keine Möglichkeit haben aus dem zugewiesenen VLAN auszubrechen. Ein Zugriff (oder Angriff) auf einen anderen Rechner, der nicht zum eigenen VLAN gehört kann daher bereits durch den Netzwerkswitch verhindert werden. Wenn eine VLAN übergreifende Kommunikation notwendig ist, können hierfür explizit Routen eingerichtet werden.

Insbesondere wird darauf hingewiesen, dass die hier beschriebene VLAN-Technologie eine solche sein kann, welche an den Industriestandard IEEE 802.1Q angepasst ist und/oder mit diesem kompatibel ist.

Der Standard IEEE 802.1Q ist eine durch das IEEE genormte Priorisierungs- und VLAN-Technologie, die im Unterschied zu den älteren, nur Port-basierten VLANs, paketbasierte tagged-VLANS implementiert. Der Ausdruck "tagged" leitet sich vom englischen Ausdruck "material tags" ab.

Es handelt sich daher bei tagged-VLANS um Netze, die Netzwerkpakete verwenden, welche eine spezielle VLAN-Markierung tragen.

Insbesondere sind nämlich in den 802.1Q-Standard Datenfelder für das V-LAN-tagging definiert, die in dem Datenbereich eines Ethernetpakets eingeführt werden können.

Insofern kann das vorliegende Netzwerk in Form eines Ethernet-Kommunikationssystems ausgebildet sein.

Das hat den Vorteil, dass in der Regel auch bereits vorhandene, ältere Switches solche Pakete weiterleiten können. Das eingefügte Tag besteht in der Regel aus mehreren Feldern, beispielsweise vier Feldern mit einer Gesamtlänge von 32 Bit.

Für die Protokoll-ID werden 2 Byte, für das Prioritätenfeld 3 Bit, für Indikator des Canonicalformats 1 Bit und für VLAN-ID 12 Bit genutzt.

Zur eindeutigen Identifizierung eines VLANS wird jedem VLAN daher zunächst eine eindeutige Nummer zugeordnet. Man nennt diese Nummer VLAN-ID. Ein Erkennungsmodul, das mit der VLAN-ID = 1 ausgestattet ist, kann mit jedem anderen Gerät im gleichen VLAN kommunizieren, nicht jedoch mit einem Gerät in einem anderen VLAN, wie z. B. ID = 2, 3,....

Um zwischen den VLANS zu unterscheiden, wird nach dem Standard IEEE 802.1Q ein Ethernetframe um 4 Byte erweitert. Davon sind 12 Bit zur Aufnahme der VLAN-ID vorgesehen, sodass (ohne Ausnutzung des Canonical Formats) theoretisch 4096-2 = 4094 VLANS möglich sind.

Denkbar ist, dass die einzelnen logischen Netzwerkverbindungen entsprechend eines OPC-Standards, d. h. beispielsweise in Form von OPC UA-Verbindungen, ausgebildet sind. Insbesondere ist nämlich denkbar, dass pro Netzwerksegment über die Steuerungseinrichtung mehrere OPC UA-Endpunkte mit unterschiedlicher IP-Adresse, VLAN-ID und Priorisierung entsprechend des oben genannten Standards IEEE 802.1Q zur Verfügung stehen.

Weist nun ein Netzwerksegment, welches eindeutig, vorzugsweise eineindeutig, eine bestimmte VLAN-ID zugewiesen bekommen hat, eine höhere Priorität auf als ein, nur in logischer Hinsicht, davon unterschiedliches Netzwerksegment einer entsprechend unterschiedlichen VLAN-ID, so können die Steuerungseinrichtung und/oder der VLAN-Switch dazu vorgesehen sein, zunächst den Datenaustausch des höher priorisierten Netzwerksegments zu bevorzugen, um erst nach Abbau der diesem höher priorisierten Netzwerksegment zugeordneten Aufgaben eine Abarbeitung des tiefer priorisierten Netzwerksegments zu erlauben.

Mit anderen Worten gilt generell: Zuordnung und Konfiguration der OPC UA Endpunkte zu einem bestimmten Netzwerksegment entsprechend der VLAN-ID und Zuordnung einer Priorität, entsprechend der Priorität des entsprechenden VLANs.

Gemäß zumindest einer Ausführungsform sind jedem Sensor und/oder jeder Verarbeitungseinheit zumindest eine VLAN-ID zugeordnet und jedem Netzwerksegment ist wiederum zumindest eine, beispielsweise genau eine, eindeutig, vorzugsweise eineindeutig, VLAN-ID zugeordnet, wobei über jede der VLAN-IDs zumindest ein Steuerelement ansteuerbar ist. Gemäß zumindest einer Ausführungsform umfasst zumindest eine Vorrichtung zumindest einen Temperatursensor, wobei der Temperatursensor eine Umgebungstemperatur und/oder eine Temperatur eines Sensors misst und an die Verarbeitungseinheit einer Vorrichtung und/oder an die zentrale CPU weiterleitet.

Gemäß zumindest einer Ausführungsform ermittelt die zentrale CPU einen Auslastungsgrad (CPU-Last und/oder Speicherverbrauch) von zumindest einer Verarbeitungseinheit, wobei bei Überschreiten einer Grenztemperatur der Verarbeitungseinheit und/oder zumindest der dieser Verarbeitungseinheit zugeordneten Sensor diese(r) in seiner Leistung zumindest teilweise gedrosselt oder ganz abgeschaltet wird

Gemäß zumindest einer Ausführungsform ist der Sensor zusätzlich ein kapazitiver Drucksensor, wobei die Verarbeitungseinheit zusätzlich dazu eingerichtet und dafür vorgesehen ist, eine durch äußeren Druck verursachte Kapazitätsänderung des Kondensators zu messen und/oder zu speichern.

Grundsätzlich handelt es sich bei einem kapazitiven Sensor also um einen Sensor, welcher auf Basis der Veränderung der elektrischen Kapazität eines einzelnen Kondensators oder eines Kondensatorsystems arbeitet. Die Beeinflussung der Kapazität durch die zu erfassende Größe kann dabei auf verschiedene Arten, erfolgen die primär durch den Verwendungszweck bestimmt ist.

Ein kapazitiver Sensor basiert unter anderem darauf, dass zwei Elektroden, einer davon kann die zumessende Oberfläche sein, die "Platten" eines elektrischen Kondensators bilden, dessen Kapazität oder Kapazitätsänderung gemessen wird, die folgendermaßen beeinflusst werden kann:
- Eine Platte wird durch den zumessenden Effekt verschoben und/oder verformt, wodurch sich der Plattenabstand und damit die elektrische messbare Kapazität ändern.
- Die Platten sind starr und die Kapazität an Sich ändert sich dadurch, dass ein elektrisch leitendes Material oder ein Dielektrikum in unmittelbare Nähe gebracht wird.
- Die wirksame Plattenfläche ändert sich, indem die Platten wie bei einem Drehkondensator gegeneinander verschoben werden.

Um auch kleine Veränderungen besser detektieren zu können kann die eigentliche Messelektrode häufig mit einer Schirmelektrode umgeben sein, die den inhomogenen Randbereich des elektrischen Feldes von der Messelektrode abschirmt, dadurch ergibt sich zwischen Messelektroden üblicherweise geerdeter Gegenelektrode eine annähernd paralleles elektrisches Feld mit der bekannten Charakteristik eines idealen Plattenkondensators.

Ein kapazitiver Drucksensor ist insbesondere ein solcher bei dem die Kapazitätsänderung infolge des Durchbiegens einer Membran und der resultierenden Änderung des Plattenabstands als Sensoreffekt ausgewertet wird. Zum Beispiel handelt es sich bei der Membran um das oben genannte Dielektrikum oder aber um die einzelnen Kondensatorelektroden welche insbesondere in Form einer Platte ausgeführt sein können. Mit anderen Worten ist in einer derartigen Ausführungsform in neuartiger Art und Weise ein kapazitiver Feuchtesensor mit einem kapazitiven Drucksensor kombiniert, jedoch ohne dass diese Bauteile voneinander getrennte Elemente oder zwei separate Sensoren bildeten, sondern es handelt sich bei vorliegender Ausführungsform um ein "Two in One"-Konzept, in welchem der gleiche Sensor sowohl als Feuchtesensor, als auch als Drucksensor fungiert.

Gemäß zumindest einer Ausführungsform handelt es sich bei dem Trägermaterial um einen Webstoff, insbesondere in welchem elektrische Leiterbahnen zur elektrischen Kontaktierung des Sensors und der Verarbeitungseinheit eingewoben sind.

Bei einem Webstoff handelt es sich im Sinne der Erfindung daher um ein Gewebe, welches manuell oder maschinell auf Basis von einzelnen Fäden gewebt wurde.

Die elektrischen Leiterbahnen können in einem Gewebe daher zusätzlich neben den üblichen Fasern und Gewebesträngen integriert sein oder aber einzelne Gewebestränge welche das Gewebenetz ausbilden ersetzen.

Je nach Abstand und Eigenschaften der einzelnen Fäden (hochgedreht, bauschig, usw.) können ganz lockere Gewebe, wie Verbandgewebe oder Dichtegewebe wie Brokatstoff entstehen. Längselastisch werden Gewebe durch, als Kettenfäden eingesetzte Gummifäden (mehr Bändern verwendet) oder Kräusel- und Bauschgarne verwendet. Sie werden gespannt, verarbeitet und ziehen sich im Ruhezustand zusammen. Bauschgarne bestehen aus texturierten, also gekräuselten synthetischen Fasern. Die Kräuselung verändert die Eigenschaften der synthetischen Fasern. Die darauf gesponnenen Garne sind sehr elastisch und voluminös und haben eine gute Wärmedämmung.

Zum Beispiel kann das Trägermaterial Teil eines Bezugstoffes eines Sitzes, insbesondere eines Fahrzeugsitzes oder eines Bürostuhls, sein. Insofern kann der Sensor vorzugsweise jedoch die gesamte Vorrichtung, auf dem Bezugsstoff eines solchen Sitzes aufgebracht oder in einen solchen integriert sein.

Zum Beispiel ist die Verarbeitungseinheit dazu eingerichtet und dafür vorgesehen, die einzelnen Feuchte- sowie Druckwerte zu erfassen und aus einer Kombination der einzelnen Feuchte- und Druckwerte zumindest einen jeweiligen Kennwert zu ermitteln, aus welchem ableitbar ist, welches Individuum (mit Gewicht und/oder Größe) gerade den Fahrzeugsitz besetzt.

Zum Beispiel kann aus der Druckmessung durch die Verarbeitungseinheit ein Gewicht der jeweiligen Person abgeleitet und festgestellt werden. Auch kann die jeweilige Feuchtigkeit, welche die jeweilige Person an den Sensor abgibt gemessen werden, wobei der jeweilige Kennwert zum Beispiel ein Produkt aus dem relativen Feuchtigkeitswert mal der von der Verarbeitungseinheit ermittelten Belastungsgewicht ist.

Überschreitet ein derartiger Kennwert einen entsprechenden Grenzwert kann die Verarbeitungseinheit insbesondere mittels einer Anbindung an die Elektronik des Fahrzeugs, eine Warnung aussprechen. Diese Warnung kann dahingehend lauten, dass der Sitz überbelegt ist oder der Fahrer zu stark schwitzt. Diese Warnung kann jedoch auch ersetzt werden durch eine entsprechende Anzeige dahingehend welcher Belegungstyp den Sitz nutzt. Bei einem Belegungstyp kann es sich um eine Gewichtsklassifikation eines jeweiligen Benutzers handeln, oder aber auch darum Handeln ob es sich bei dem Benutzer um ein Tier, einen Menschen oder auch um eine Sache handelt. Vorzugsweise ist daher die Verarbeitungseinheit in eine Anzeigenelektronik des Fahrzeugs integrierbar, zumindest jedoch mit einer solchen verbindbar.

Hierzu ist denkbar, dass die Verarbeitungseinheit sich zum Beispiel mittels Bluetooth oder einer sonstigen Wireless Verbindung mit einer Empfangseinheit des Fahrzeugs verbindet und der jeweilige Kenn- oder Grenzwert und/oder die jeweilige Warnung und/oder die jeweilige Identifikation des Benutzers auf einem Display des Fahrzeugs wiedergegeben werden.

Alternativ oder zusätzlich ist vorstellbar, dass diese einzelnen Werte und/oder Identifikationen auch extern abrufbar und/oder extern darstellbar sind. Zum Beispiel kann das Auto auf eine Überbelegung hin von einem externen Controller überwacht werden.

Zum Beispiel kann mittels einer Datenverbindung die Verarbeitungseinheit mit einer Auslöseinheit eines Airbags in Verbindung stehen, sodass die Verarbeitungseinheit auch die Auslöseinheit steuern und/oder regeln kann, insbesondere in Bezug auf einen Auslösezeitpunkt des Airbags. Zusätzlich und/oder alternativ ist es möglich, dass die Verarbeitungseinheit eine Controllereinheit des Airbags mit Daten zum Beispiel im Hinblick auf einen Belegungstyp, Position und/oder Gewicht eines Benutzers des Fahrzeugsitzes versorgt.

Diese Daten können dazu führen, dass der Auslösezeitpunkt und die Auslösereihenfolge des Airbags auf den Benutzer angepasst sind, sodass ein Personenschaden an dem Benutzer vermieden wird.

Gemäß zumindest einer Ausführungsform ist zumindest eine Elektrode und/oder dielektrische Schicht auf dem Trägermaterial oder auf einer auf dem Trägermaterial angeordneten, insbesondere wasserundurchlässigen Schicht aufgedruckt oder mittels eines Dünnschichtverfahrens aufgebracht.

Dies heißt, dass zumindest ein Element, vorzugsweise sowohl die Elektrode als auch die dielektrische Schicht, auf dem Trägermaterial oder einer zwischen dem Sensor und dem Trägermaterial aufgebrachten vorzugsweise elektrisch nicht leitfähigen, weiter vorzugsweise wasserundurchlässigen Schicht mittels eines Druckverfahrens aufgedruckt.

Bei dem Druckverfahren kann es sich zum Beispiel um ein Inkjetverfahren handeln.

Zum Beispiel ist die Verarbeitungseinheit in der gleichen Weise wie der Sensor auf das Trägermaterial aufgebracht. Hierzu ist vorstellbar, dass auch die Verarbeitungseinheit, zumindest jedoch eine, insbesondere leitende, Schicht der Verarbeitungseinheit auf das Trägermaterial zum Beispiel aufgedruckt ist. Die Datenkommunikation zwischen der Verarbeitungseinheit und dem Sensor kann dann über die oben genannten Leiterbahnen entstehen. Diese Leiterbahnen können zumindest teilweise, vorzugsweise jedoch vollständig, in den Webstoff eingewoben sein oder sogar einzelne Fasern des Webstoffs selbst ausbilden.

Zum Beispiel ist zumindest eine Elektrode flächig ausgeführt. Das heißt, dass eine Dicke der Elektrode im Vergleich zu deren Flächenausdehnung vernachlässigbar ist. Eine solche Elektrode kann daher insbesondre mittels eines Druckverfahrens hergestellt werden.

Alternativ hierzu kann eine Dicke zumindest einer Elektrode höchstens 5 mm betragen. Hierzu kann das Druckverfahren mehrmals angewandt werden, sodass zumindest zwei, vorzugsweise jedoch dann mehr, Einzeldruckschichten übereinander gestapelt werden.

Des Weiteren kann die Elektrode auch mittels eines 3D-Druckverfahrens auf dem Trägermaterial angeordnet sein.

### 1. Das FDM-Verfahren (Fused Deposition Modeling)

Alternativbezeichnungen: Fused Filament Fabrication (FFF), Fused Layer Modeling (FLM) Das Verfahren bezeichnet schichtweises Auftragen (Extrusion) eines Materials durch eine heiße Düse. Das Verbrauchsmaterial befindet sich in Form eines langen Drahts (sog. Filament) auf einer Rolle und wird durch die Fördereinheit in einen Druckkopf geschoben, dort eingeschmolzen und auf einem Druckbett ausgebracht. Druckkopf und/oder Druckbett sind dabei in drei Richtungen beweglich. So können Kunststoffschichten schrittweise aufeinander aufgebracht werden.

### 2. Das SLS Verfahren (Selektives Lasersintern)

Im Unterschied zum Sinterverfahren, bei dem Stoffe in Pulverform unter Hitzeeinwirkung miteinander verbunden werden, geschieht dies beim SLS-Verfahren selektiv durch einen Laser (alternativ auch Elektronenstrahl oder Infrarotstrahl). Es wird also nur ein bestimmter Teil des Pulvers miteinander verschmolzen.

Dazu wird stets eine dünne Pulverschicht von der Beschichtungseinheit auf dem Druckbett ausgebracht. Der Laser (oder andere Energiequelle) wird nun punktgenau auf einzelne Stellen der Pulverschicht ausgerichtet, um die erste Schicht der Druckdaten auszubilden. Hierbei wird das Pulver an- oder aufgeschmolzen und verfestigt sich anschließend wieder durch geringfügiges Abkühlen. Das nicht aufgeschmolzene Pulver bleibt um die gesinterten Bereiche herum liegen und dient als Stützmaterial. Nachdem eine Schicht verfestigt ist, senkt sich das Druckbett um den Bruchteil eines Millimeters ab. Die Beschichtungseinheit fährt nun über das Druckbett und bringt die nächste Pulverschicht aus. Anschließend wird die zweite Schicht der Druckdaten durch den Laser (oder eine andere Energiequelle) gesintert. So entsteht schichtweise ein dreidimensionales Objekt.

### 3. Three-Dimensional Printing (3DP)

Das 3DP-Verfahren funktioniert sehr ähnlich wie das selektive Lasersintern, doch anstelle einer gerichteten Energiequelle verfährt ein Druckkopf über das Pulver. Dieser gibt winzige Tröpfchen von Bindemittel auf die zugrunde liegenden Pulverschichten ab, die so miteinander verbunden werden. Ansonsten ist dieses Verfahren dem SLS-Verfahren gleich.

### 4. Stereolithographie (SLA)

Anstelle eines Kunststoffdrahts oder Druckmaterials in Pulverform kommen beim Stereolithographie-Verfahren flüssige Harze, sog. Photopolymere, zum Einsatz. Sie werden schichtweise durch UV-Strahlung verhärtet und erzeugen so dreidimensionale Objekte. Dafür wird die Bauplattform im Harzbecken schrittweise abgesenkt. Es gibt auch Varianten (sog. Polyjet-Verfahren) ohne ein ganzes Becken mit flüssigem Harz. Dafür wird ein Epoxidharz tröpfchenweise aus einer Düse aufgebracht und durch einen UV-Laser sofort ausgehärtet.

### 5. Laminated Object Manufacturing (LOM)

Alternativbezeichnung: Layer Laminated Manufacturing (LLM)

Das Verfahren basiert weder auf chemischen Reaktionen, noch auf einem thermischen Prozess. Es wird dabei mit einem trennenden Werkzeug (z.B. einem Messer oder Kohlendioxidlaser), einer Folie oder einer Platte (z.B. Papier) an der Kontur geschnitten und schichtweise aufeinander geklebt. So entsteht durch Absenken der Bauplattform ein Schichtobjekt aus geklebten, übereinanderliegenden Folien.

Eine oder mehrere wasserundurchlässige Schichten und/oder auch die Feuchteschicht können in derselben Art und/oder Dicke wie die Elektrode aufgebracht werden.

Gemäß zumindest einer Ausführungsform bedeckt die Feuchteschicht den Kondensator vollständig.

Dies kann heißen, dass die Feuchteschicht, nach außen, das heißt in der Querrichtung den Sensor nach außen abgrenzt und abschließt, sodass der Sensor zwischen der Feuchteschicht und dem Trägermaterial angeordnet ist.

Gemäß zumindest einer Ausführungsform weist der Sensor zumindest einen weiteren Kondensator auf, welcher in der Querrichtung unter oder über dem Kondensator angeordnet und durch eine weitere wasserundurchlässige Schicht beabstandet von dem Kondensator auf oder unter dieser weiteren wasserundurchlässigen Schicht angeordnet ist, sodass ein Kondensatorenstack entsteht.

Der weitere Kondensator kann in der gleichen Weise wie der Kondensator aufgebaut sein und ebenso in einer gleichen Weise wie der Kondensator auf die weitere wasserundurchlässige Schicht angeordnet sein.

Mittels eines derartigen Kondensatorenstacks kann die Sensorik ganz besonders einfach verfeinert werden nämlich insofern, als dass denkbar ist das bei zwei den Kondensatorstack ausbildenden Sensoren beide Sensoren die gleichen Aufgaben verrichten, jedoch durch die einzelnen Sensoren jeweilige Messwerte ermittelt werden, die zusammen genommen auf einen Mittelwert schließen lassen. Zum Beispiel wird von jedem der beiden Sensoren jeweils die (relative) Feuchtigkeit der Umgebung gemessen wobei aus diesen beiden Messwerten dann der Feuchtigkeitsmittelwert ermittelt wird. Gleiches kann entsprechend mit der Druckmessung geschehen, sodass die Genauigkeit der gesamten Messung insbesondere einer Kombination der Messungen von (relativer) Feuchtigkeit und dem jeweiligen Druck besonders genau ausgestaltet werden kann.

Gemäß zumindest einer Ausführungsform bildet die wasserundurchlässige Schicht und/oder die weitere wasserundurchlässige Schicht die dielektrische Sicht zumindest teilweise selbst aus.

Dies kann heißen, dass ein anstatt der separaten Positionierung einer dielektrischen Schicht neben der wasserundurchlässigen Schicht und/oder neben der weiteren wasserundurchlässigen Schicht, diese dielektrische Schicht selbst durch die wasserundurchlässige Schicht und/oder die weitere wasserundurchlässige Schicht gebildet ist.

Eine derartige Erzeugung der dielektrischen Schicht durch die wasserundurchlässige(n) Schicht(en) bildet daher ein besonders einfaches und kostengünstiges Herstellungsverfahren zu einer kostengünstigen Vorrichtung.

Davon abgesehen kann grundsätzlich vorgesehen sein die Elektroden, die dielektrische Schicht und die wasserundurchlässige Schicht(en) derart zueinander anzuordnen, dass ein elektrischer Kurzschluss in jedem Fall verhindert ist.

Gemäß zumindest einer Ausführungsform beträgt eine maximale Dicke der Feuchteschicht wenigstens 30% und höchstens 80% der maximalen Dicke der wasserundurchlässigen Schicht und/oder der maximalen Dicke der weiteren wasserundurchlässigen Schicht.

Dies stellt nicht nur einen besonders flach gebauten Sensor sicher, sondern gewährleistet auch eine besonders schnelle Reaktionszeit auf Feuchtigkeitsveränderungen. Die von außen auf die Feuchteschicht einwirkende Feuchtigkeit muss daher keine großen Strecken zu dem Dielektrikum durchwandern.

Des Weiteren betrifft die vorliegende Erfindung ein Verfahren zur Messung von Druck und/oder Feuchtigkeit wobei insbesondere angemerkt sei, dass alle für die obig beschriebene Vorrichtung offenbarten Merkmale auch für das hier beschriebene Verfahren offenbart sind und umgekehrt.

Gemäß zumindest einer Ausführungsform umfasst das Verfahren zu Messung von Druck und/oder Feuchtigkeit zunächst einen ersten Schritt mittels welchem zumindest ein Messsystem, insbesondere nach zumindest einem der vorhergehenden Ansprüche, bereitgestellt wird, wobei durch das Messsystem zumindest ein Sensor zu Messung von Druck und/oder Feuchtigkeit bereitgestellt wird, wobei der Sensor zumindest einen Kondensator mit zumindest zwei Elektroden, welche, insbesondere in einer horizontalen Richtung entlang eines und auf einem, insbesondere flexiblem, Trägermaterial zueinander angeordnet sind aufweist, wobei zwischen den Elektroden zumindest eine dielektrische Schicht angeordnet ist.

Erfindungsgemäß ist auf einer dem Trägermaterialabgewandten Seite zumindest einer Elektrode und/oder der dielektrischen Schicht zumindest stellenweise, zumindest eine, zumindest teilweise flüssigkeitsdurchlässige und/oder flüssigkeitsabsorbierende Feuchteschicht angeordnet, wobei somit die zumindest eine Elektrode und/oder die dielektrische Schicht in einer Querrichtung zwischen dem Trägermaterial und der Feuchteschicht angeordnet sind, sodass sich eine Kapazität durch die auf die dielektrische Schicht zumindest teilweise treffende Flüssigkeit zumindest teilweise verändert, wobei eine Verarbeitungseinheit diese Änderung misst und/oder speichert, sodass ein kapazitiver Feuchtesensor entsteht.

Dabei weist das oben beschrieben Verfahren die gleichen Vorteile und vorteilhaften Ausgestaltungen wie die obig beschriebene Vorrichtung auf.

Im Folgenden wird die hier beschriebene Erfindung anhand zweier Ausführungsbeispiele und den dazugehörigen Figuren näher beschrieben.

Gleiche oder gleichwirkende Bestandteile sind mit den gleichen Bezugszeichen versehen.

Die Figur 1A zeigt ein Verfahren zum Überwachen eines Fahrers eines Fahrzeugs mittels eines Messsystems.

Die Figur 1B zeigt in einem Zeitdiagrammverlauf, Messzeitpunkte innerhalb eines Messintervalls.

Die Figuren 2A bis 2C zeigen ein Ausführungsbeispiel eines hier beschriebenes und erfindungsgemäßes Messsystems.

In der Figur 3 ist in einem ersten Ausführungsbeispiel eine erfindungsgemäße Vorrichtung zur Messung von Druck und/oder Feuchtigkeit gezeigt.

In der Figur 4 ist einer schematisch perspektivischen Ansicht eine in Bezug auf die Schichtenordnung dargestellte Explosionszeichnung dargestellt.

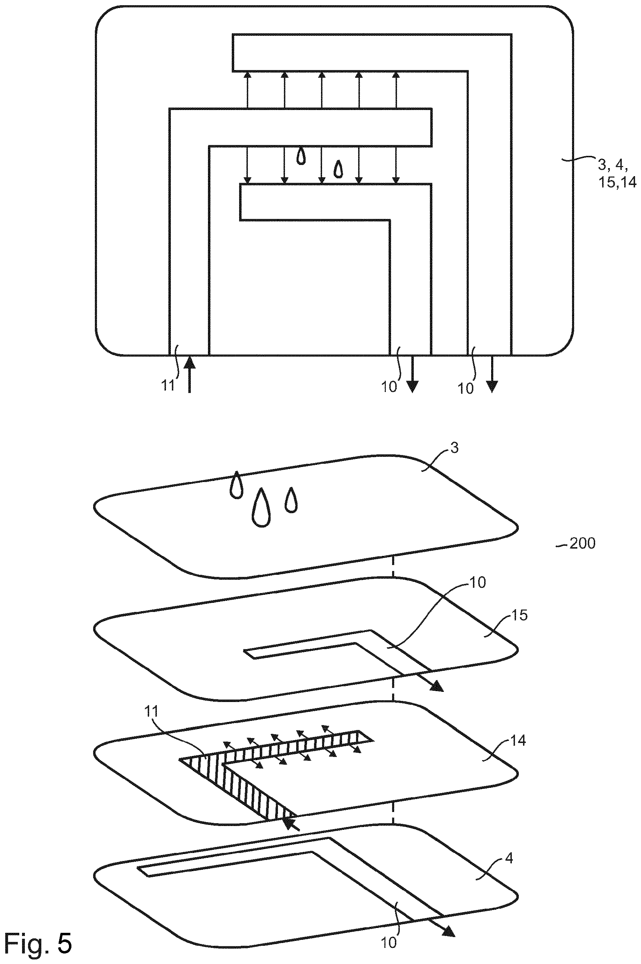

In der Figur 5 ist ein weiteres Ausführungsbeispiel einer hier beschriebenen Vorrichtung gezeigt.

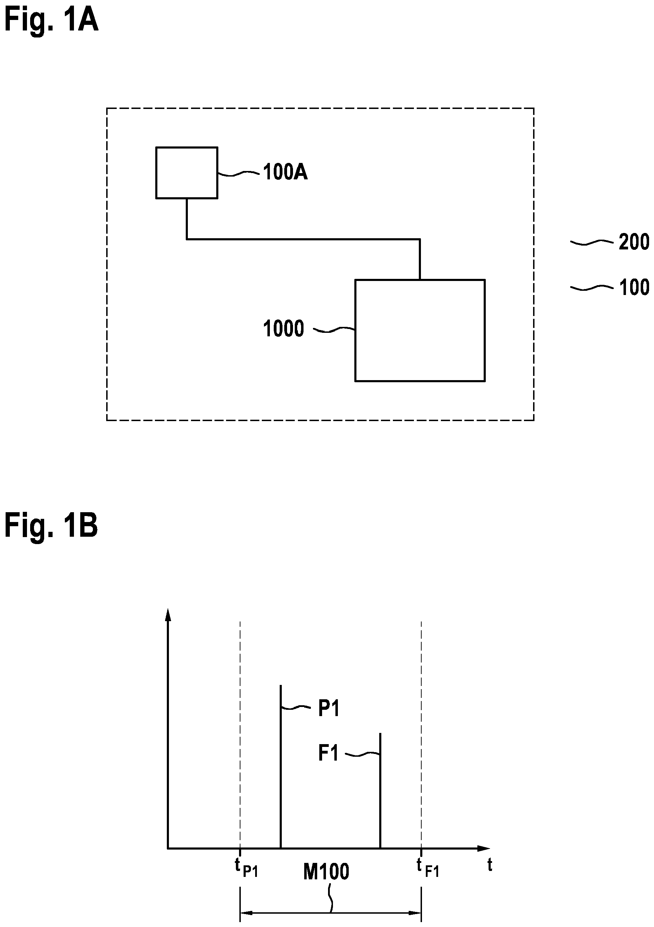

Die Figur 1A zeigt ein Verfahren 200 sowie eine Vorrichtung 100 zum Überwachen eines Fahrers eines Fahrzeugs mittels eines Messsystems 1000.

Dabei umfasst das Verfahren 200 einen ersten Schritt, wonach ein Messsystem 1000 zur Messung von Druck und/oder Feuchtigkeit bereitgestellt wird, wobei das Messsystem 1000 mit mindestens einem Fahrzeugelement 100A gekoppelt oder mit mindestens einem integriert verbaut ist, und das Messsystem 1000 zumindest einen Sensor 1 zur Messung eines, vorzugsweise nur eines, Stresslevels, des Fahrers aufweist, sodass durch den Sensor 1 Druck und/oder Feuchtigkeit gemessen wird.

Anschließend werden die gemessenen Stresslevelwerte, also die Druck- und/oder Feuchtigkeitswerte, an eine Verarbeitungseinheit 5 des Messsystems 1000 weitergeleitet, wobei in einem weiteren Schritt das Stressniveaus des Fahrers basierend auf den Stresslevelwerten erkannt wird.

In einem weiteren Schritt wird eine Aktion basierend auf der Erkennung durch das Messsystem 1000 ausgewählt, wobei die Aktion ausgewählt ist aus einer Liste bestehend aus: Einrichten eines Anrufs zu einer entfernten Unterstützungsstelle, Übertragen der Stresslevelwerte zu einer entfernten Unterstützungsstelle, Erzeugen eines akustischen Alarms und Erzeugen eines visuellen Alarms, Anpassung einer Lautstärke von Lautsprechern in dem Fahrzeug, Anpassen einer Sitzposition eines Fahrzeugsitzes des Fahrzeugs, sowie Anzeige von Pausenempfehlungen an den Fahrer.

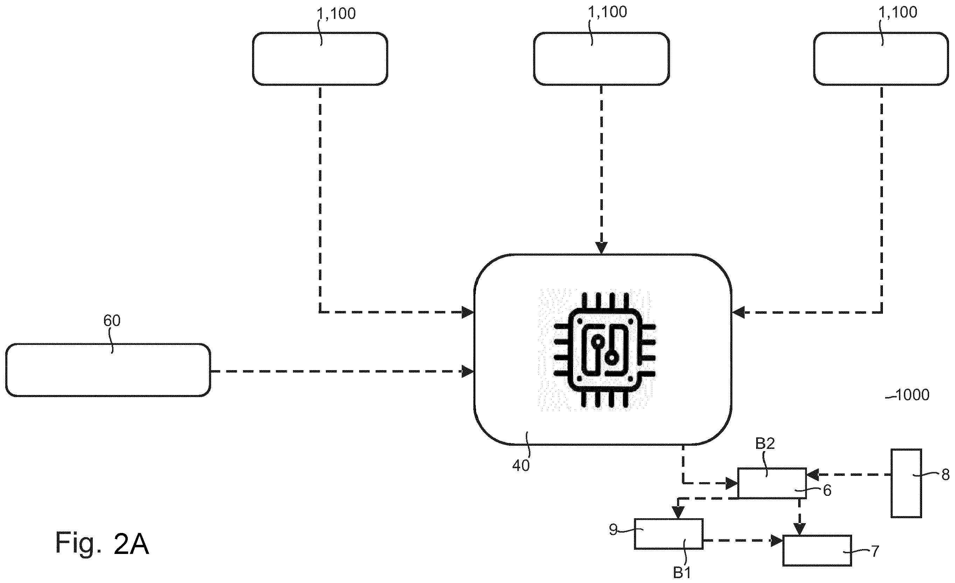

Dabei sind in einem Speicher einer CPU 40 (zu sehen in der Figur 2A) Grenzwerte von Druck und/oder Feuchtigkeit hinterlegt, wobei die jeweils zeitdiskret oder kontinuierlich gemessenen Druck- und Temperaturwerte mit den in dem Speicher der CPU 40 hinterlegten Werten verglichen wird, wobei bei einer Überschreitung zumindest einer dieser Werte (Feuchtigkeit und Druck) von der CPU 40 eine Aktion zur Ausführung bestimmt wird.

Die Figur 1B zeigt in einer möglichen Ausführungsform, dass innerhalb eines vorbestimmten Messzeitintervalls M100 ein Druck P1 und eine Feuchtigkeit F1 jeweils zu verschiedenen Zeitpunkten gemessen werden. Möglich ist jedoch auch eine zeitgleiche Messung beider Werte.

Der Figur 2A ist in einem Ausschnitt ein schematischer Aufbau eines hier beschriebenen erfindungsgemäßen Messsystems 1000 gezeigt. Erkennbar ist eine Verarbeitungseinheit 5, welche in Datenkommunikation mit einer Mehrzahl von Sensoren 1 ist. Die Verarbeitungseinheit 5 bildet zusammen mit den Sensoren 1 eine Vorrichtung 100. Die von den einzelnen Sensoren 1 gemessen Feuchtigkeits- und/oder Druckwerte werden eine zentrale CPU 40 gesendet um dort gespeichert und/oder weiterverarbeitet zu werden. Zudem ist ein Temperatursensor 60, welcher eine Umgebungstemperatur und/oder eine Temperatur des Sensors 1 misst und an die Verarbeitungseinheit 5 der Vorrichtung 100 und/oder an die zentrale CPU 40 weiterleitet.

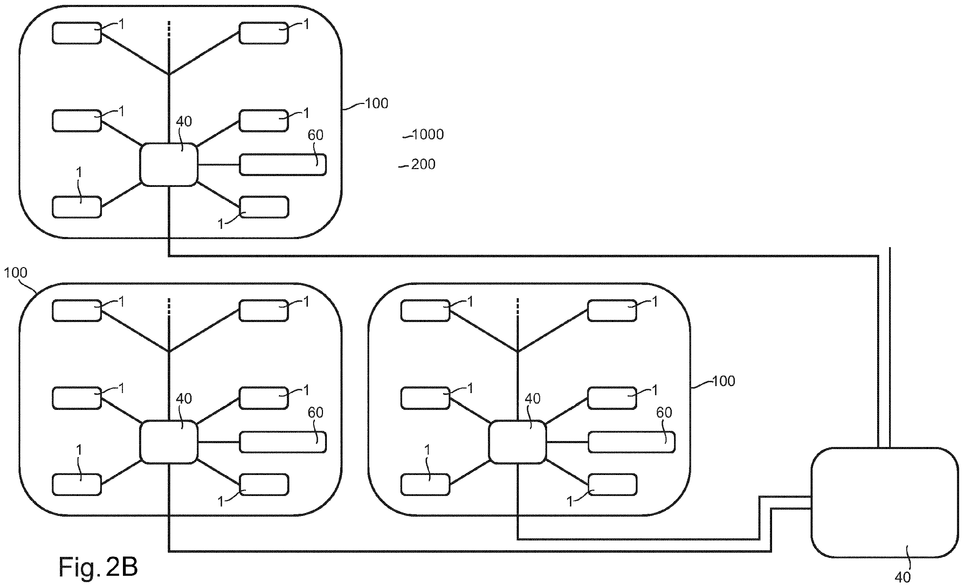

Die Figur 2B zeigt schematisch das gesamte Messsystem 1000, mit einer Mehrzahl an Sensorgruppen, welche durch die einzelnen Vorrichtungen 100 zur Messung von Druck und/oder Feuchtigkeit gebildet sind und welche jeweils eine Verarbeitungseinheit 5 zeigen. Jeder Verarbeitungseinheit 5 ist daher eine Mehrzahl an Sensoren 1 zugeordnet.

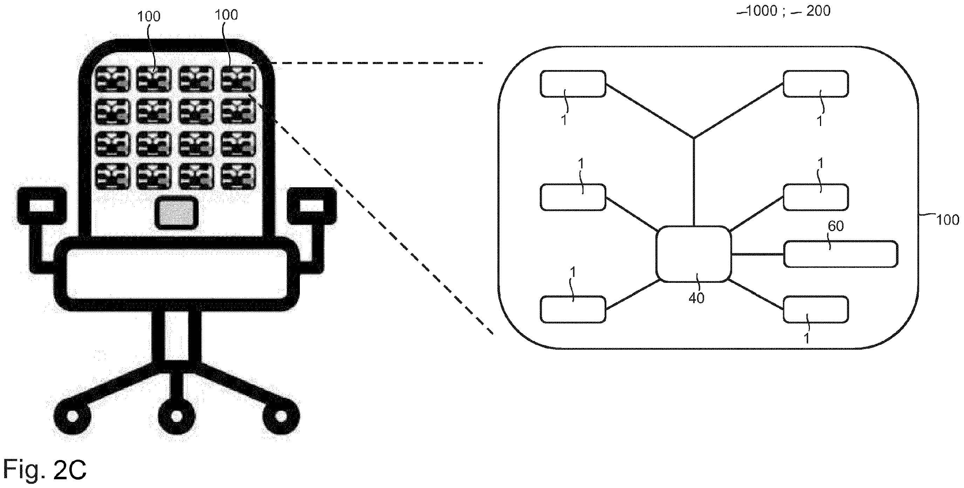

Die Figur 2C zeigt schematisch eine Verbauung und Integration des Messsystems 1000 in einen Stuhl, insbesondere in einen Bürostuhl.

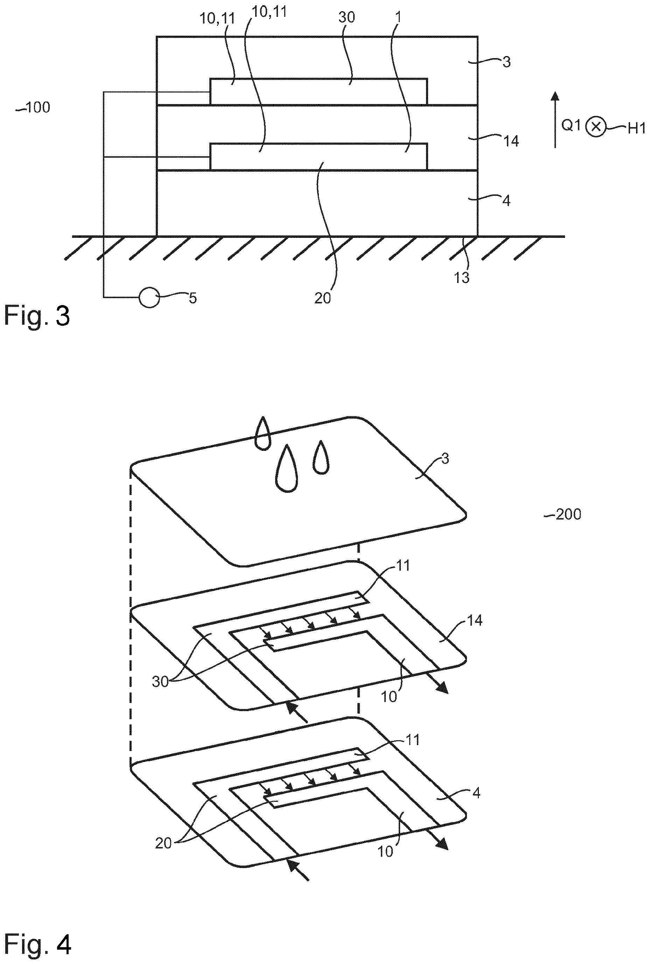

Wie nun der Figur 3 entnommen werden kann, ist dort eine Vorrichtung 100 zur Messung von Druck und/oder Feuchtigkeit im Detail gezeigt.

Beispielhaft ist dort ein Sensor 1 dargestellt, wobei der Sensor 1 einen Kondensatorstack zeigt mit einem Kondensator 20, sowie einem Kondensator 30, wobei die einzelnen Elektroden 10, 11 der Kondensatoren 20, 30 in der horizontalen Richtung H1 übereinander angeordnet sind, wobei alternativ hierzu jedoch selbstverständlich eine Anordnung der einzelnen Elektroden 10, 11 eines einzelnen Kondensators 20, 30 in der Querrichtung Q1 welche senkrecht zur horizontalen Richtung H1 verläuft und damit auch senkrecht zur Haupterstreckungsrichtung des dort gezeigten Sensors 1 verlaufen oder angeordnet sein können.

Die einzelnen Elektroden 10, 11 sind auf einem Trägermaterial 13 angeordnet. Bei dem Trägermaterial 13 kann es sich insbesondere um einen Webstoff, insbesondere um einen flexiblen Webstoff handeln.

Auf dem Trägermaterial 13 ist eine wasserundurchlässige Schicht 4 angeordnet, wobei auf dieser wasserundurchlässigen Schicht 4 die beiden Elektroden 10, 11 des Kondensators 20 in der horizontalen Richtung H1 aufgedruckt sind.

Die Elektroden 10, 11 des Kondensators 20 sind vollständig von einer weiteren wasserundurchlässigen Schicht 14 umgeben. Auf dieser wasserundurchlässigen Schicht 14 ist in der gleichen Art und Weise der weitere Kondensator 30 mit entsprechenden Elektroden 10, 11 aufgedruckt. Zudem sind in dem vorliegenden Ausführungsbeispiel freiliegende Außenflächen der einzelnen Elektroden 10, 11 des weiteren Kondensators 30 vorzugsweise vollständig von einer wasserdurchlässigen und/oder wasserabsorbierenden Feuchteschicht 3 umgeben.

Über diese Feuchteschicht 3 kann Wasser auf eine dielektrische Schicht 4 treffen, welche vorliegend in der horizontalen Richtung H1 zwischen den jeweiligen Elektroden 10, 11 eines Kondensators 20, 30 angeordnet ist.

Im vorliegenden Ausführungsbeispiel der Figuren 3 und 4 bildet die wasserundurchlässige Schicht 4 selbst eine dielektrische Schicht 4 des Kondensators 20 auf. Selbiges gilt für die weitere wasserundurchlässige Schicht 14 in Bezug auf den weiteren Kondensator 30. Durch Auftreffen und Durchdringen der Feuchtigkeit über die Feuchteschicht 3 werden die dielektrischen Eigenschaften insbesondere der dielektrischen Schicht 4 des weiteren Kondensators 30 verändert.

Darüber hinaus ist eine Verarbeitungseinheit 5 erkennbar, welche in datentechnischer Verdingung mit den beiden Kondensatoren 20, 30 steht, wobei diese Verarbeitungseinheit 5 dazu eingerichtet und dafür vorgesehen ist, eine Änderung der relativen Feuchtigkeit der Umgebung und/oder der Feuchteschicht 3 zu messen.

Durch die in der Figur 4 dargestellte "stackwise"-Anordnung und dadurch, dass die weitere wasserundurchlässige Schicht 14 verhindert, dass der Kondensator 20 mit Feuchtigkeit in Kontakt kommt, kann daher vorgesehen sein, dass lediglich der weitere Kondensator 30 und dessen dielektrische Schicht 4 der Feuchtigkeit ausgesetzt ist. Hierzu kann die Verarbeitungseinheit 5 dann eine Veränderung der Kapazität des weiteren Kondensators 30 vergleichen mit der stabilen Kondensatorkapazität des Kondensators 20, sodass hierzu ein besonders einfacher Vergleich in der Veränderung der relativen Feuchtigkeit und/auch des jeweiligen Belastungsdruckes hergestellt sein kann.

Durch den in der Figur 3 dargestellten Pfeil ist auch eine Druckrichtung unter welcher der Sensor 1 mit Druck beaufschlagt wird gezeigt. Beides kann vorzugsweise durch den Sensor 1 und insbesondere durch die Vorrichtung 100 gemessen, ausgewertet und gespeichert werden. Hierzu dient insbesondere die in der Erfindung als wesentlich dargestellte Verarbeitungseinheit 5, welche zusätzlich auch entsprechende Druckwerte und insofern damit verbundene Änderungen in der Kapazität der einzelnen Sensoren 1 messen und auswerten kann, sodass die Verarbeitungseinheit 5 zusätzlich dazu eingerichtet und dafür vorgesehen ist eine durch äußeren Druck verursachte Kapazitätsänderung des Kondensators 20 und insbesondere auch des weiteren Kondensators 30 zu messen und/oder zu speichern.

Die Feuchteschicht 3 kann flexibel oder nicht flexibel ausgebildet sein. Zudem ist es möglich, dass die Feuchteschicht 3 als Webstoff ausgebildet ist. Insbesondere kann es sich um einen Webstoff handeln, welcher im einleitenden Teil der vorliegenden Anmeldung beispielhaft genannt wurde. Zudem ist es jedoch auch möglich, dass es sich bei der Feuchteschicht 3 um ein Substrat handelt welches, zum Beispiel in Form eines Epitaxie- oder eines Klebeprozesses auf den weiteren Kondensator 30 aufgebracht, zum Beispiel aufgeklebt wurde.

Die wasserundurchlässige Schicht 14 und/oder die wasserundurchlässige Schicht 15 können ebenso flexibel und nicht flexibel, insbesondere auch ebenso in Form eines Webstoffes oder eines Substrats in der gleichen Weise wie die Feuchteschicht 3 ausgebildet sein.

Zudem ist vorteilhaft denkbar, dass die Elektroden 10, 11 der beiden Kondensatoren 20, 30 auf die wasserundurchlässige Schicht 14 und die weitere wasserundurchlässige Schicht 15 in Form eines Druckprozesses zum Beispiel eines Tintenstrahldruckprozesses aufgedruckt wurden.

In der Figur 3 ist eine Explosionszeichnung gezeigt, wobei insbesondere aus der Figur 3 die jeweilige Anordnung der Elektroden 10, 11 der Kondensatoren 20, 30 hervorgeht. Erkennbar ist wiederum die durch die Pfeilrichtung dargestellte Krafteinwirkung auf den Sensor 1, sowie durch die einzelnen schematisch dargestellten Tropfen einwirkende Feuchtigkeit. Insbesondere ist wiederum erkennbar, dass die Feuchtigkeit insbesondere zwischen den Elektroden 10, 11 eindringt und auf die jeweilige wasserdurchlässige Schicht 14 einen zum Beispiel erheblichen Effekt auf die elektrische Eigenschaft hat, sodass sich die Kapazität zumindest des weiteren Kondensators 30 wie in der Figur 1 erläutert jeweils ändert.

In der Figur 4 ist in einer weiteren Ausführungsform der hier beschriebenen Erfindung gezeigt, dass der Sensor 1 aus zwei Elektroden 10, sowie einer Elektrode 11 bestehen kann. Die Elektroden 10 haben eine Polarität (vorzugsweise die gleiche Polarität), während die Elektrode 11 eine davon unterschiedliche Polarität aufweist, wobei jedoch im unteren Teilbild der Figur 3 die Explosionszeichnung des linken Teils der Figur 3 gezeigt ist und erkennbar ist, dass drei wasserundurchlässige Schichten 4, 14, 15 verwendet werden. Die Elektroden 10 können auch unterschiedliche Polaritäten und/oder elektrische Potentiale aufweisen. Auch können die Elektroden 10 miteinander elektrisch verbunden sein.

Zum Beispiel können die Elektroden 10, 11 auch jeweils eine separate Polarität und/oder ein separates elektrisches Potential aufweisen und/oder generieren. Entsprechendes kann auch für die in den hier folgenden Figuren in Bezug auf die Elektroden gelten.

Zum Beispiel ist die unterste wasserundurchlässige Schicht wiederum die wasserundurchlässige Schicht 4, die darauffolgende wasserundurchlässige Schicht 14 und die in der Querrichtung Q1 darauf angeordnete wasserundurchlässige Schicht 15 eine weitere wasserundurchlässige Schicht, wobei jeweils eine Elektrode 10, 11 auf einer separaten wasserundurchlässigen Schicht jeweils aufgebracht insbesondere aufgedruckt ist.

In dieser Stackung der einzelnen wasserundurchlässigen Schichten 4, 14 und 15 wird daher durch Zusammenführen dieser Schichten der im linken Teil der Figur 4 gezeigte Kondensator 20 erzeugt, wobei hierbei in der Querrichtung Q1, die Elektroden 10 jeweils, wie im dementsprechenden Teilbild entnommen werden kann auf unterschiedlichen Ebenen angeordnet sin.

Alternativ hierzu kann auch die Elektrode 11 zusammen mit zumindest einer der Elektroden 10 in einer gemeinsamen Ebene, das heißt auf oder in einer gemeinsamen wasserundurchlässigen Schicht 4, 14, 15 aufgebracht werden, sodass zum Beispiel nur noch die zweite der Elektroden 10 auf eine separaten wasserundurchlässigen Schicht 4, 14, 15 aufgestackt werden muss.

Grundsätzlich können daher die einzelnen Elektroden 10,11 in unterschiedlichen Ebenen in der Q1-Richtung zueinander angeordnet sein. Zum Beispiel gilt eine paarweise Zuordnung zwischen genau einer wasserundurchlässigen Schicht 4, 14, 15 mit genau einer Elektrode 10, 11.

### Bezugszeichenliste

- 1: Sensor
- 3: Feuchteschicht
- 4: dielektrische Schicht/ wasserundurchlässige Schicht
- 5: Verarbeitungseinheit
- 10: Elektrode
- 11: Elektrode
- 13: Trägermaterial
- 14: wasserundurchlässige Schicht
- 15: wasserundurchlässige Schicht
- 20: Kondensator
- 30: Kondensator
- 40: CPU
- 60: Temperatursensor
- 100: Vorrichtung
- 100A: Fahrzeugelement
- 200: Verfahren
- 1000: Messsystem

- P1: Druck
- F1: Feuchtigkeit
- H1: horizontalen Richtung
- M100: Messzeitintervall
- Q1: Querrichtung

## Patentansprüche

1. Verfahren (200) zum Überwachen eines Fahrers eines Fahrzeugs mittels eines Messsystems (1000), wobei das Verfahren (200) die folgenden Schritte umfasst:
- Bereitstellen von zumindest einem Messsystem (1000) zur Messung von Druck und/oder Feuchtigkeit, wobei das Messsystem (1000), welches mit mindestens einem Fahrzeugelement (100A) gekoppelt oder mit mindestens einem integriert verbaut ist, und das Messsystem (1000) zumindest einen Sensor (1) zur Messung eines, vorzugsweise nur eines, Stresslevels, des Fahrers aufweist, sodass durch den Sensor (1) Druck und/oder Feuchtigkeit gemessen wird;
- anschließendes Weiterleiten der gemessenen Stresslevelwerte, also die Druck- und/oder Feuchtigkeitswerte, an eine Verarbeitungseinheit (5) des Messsystems (1000);
- Erfassen und Erkennen eines Stressniveaus des Fahrers basierend auf den Stresslevelwerten;
- Bestimmen einer ausgewählten Aktion basierend auf der Erkennung durch das Messsystem (1000), wobei die Aktion ausgewählt ist aus einer Liste bestehend aus: Einrichten eines Anrufs zu einer entfernten Unterstützungsstelle, Übertragen der Stresslevelwerte zu einer entfernten Unterstützungsstelle, Erzeugen eines akustischen Alarms und Erzeugen eines visuellen Alarms, Anpassung einer Lautstärke von Lautsprechern in dem Fahrzeug, Anpassen einer Sitzposition eines Fahrzeugsitzes des Fahrzeugs, sowie Anzeige von Pausenempfehlungen an den Fahrer, wobei
- innerhalb eines Messzeitintervalls (M100) zuerst der Druck und/oder die Temperatur und erst danach die Feuchtigkeit und/oder die Temperatur gemessen wird, wobei innerhalb jedes Messzeitintervalls (M100) nur einmal der Druck und nur einmal die Feuchtigkeit gemessen werden

2. Verfahren (200) nach Anspruch 1,
**dadurch gekennzeichnet, dass**
durch den Sensor (1) nur der Druck und die Feuchtigkeit gemessen wird, um ein Stresslevel des Fahrers festzustellen.

3. Verfahren (200) nach Anspruch 1 oder 2,
**dadurch gekennzeichnet, dass**
der Sensor (1) in einem Lenkrad und/oder einem Joystick und/oder einem Fahrzeugsitz eines Fahrzeugs verbaut ist, sodass der Fahrer das Lenkrad und/oder den Joystick und/oder den Fahrzeugsitz unmittelbar berührt.

4. Verfahren (200) nach zumindest einem der vorhergehenden Ansprüche,
**dadurch gekennzeichnet, dass**
in einem Speicher einer CPU (40) Grenzwerte von Druck und/oder Feuchtigkeit hinterlegt sind, wobei die jeweils zeitdiskret oder kontinuierlich gemessenen Druck- und Temperaturwerte mit den in dem Speicher der CPU (40) hinterlegten Werten verglichen wird, wobei bei einer Überschreitung zumindest einer dieser Werte (Feuchtigkeit und Druck) von der CPU (40) eine Aktion zur Ausführung bestimmt wird.

5. Verfahren (200) nach zumindest einem der vorhergehenden Ansprüche,
**dadurch gekennzeichnet, dass**
in einem Speicher der CPU (40) Faktorgrenzwerte aus Druck und Feuchtigkeit hinterlegt sind, wobei die jeweils zeitdiskret oder kontinuierlich gemessenen Druck- und Temperaturwerte mit den in dem Speicher der CPU (40) hinterlegten Werten verglichen wird, wobei bei einer Überschreiten zumindest einer dieser Werte (Feuchtigkeit und Druck) von der CPU (40) eine Aktion zur Ausführung bestimmt wird, wobei der Faktorgrenzwert als ein Faktor des jeweiligen Druck- und Feuchtigkeitswerts definiert ist, insbesondere wobei beide Werte zum gleichen Zeitpunkt von dem Sensor (1) gemessen werden.

6. Verfahren (200) nach zumindest einem der vorhergehenden Ansprüche,
**dadurch gekennzeichnet, dass**
innerhalb eines vorbestimmten Messzeitintervalls (M100) der Druck und die Feuchtigkeit jeweils zu verschiedenen Zeitpunkten gemessen wird.

7. Verfahren (200) nach zumindest einem der vorhergehenden Ansprüche,
**dadurch gekennzeichnet, dass**
ein zeitlicher Abstand von zwei unmittelbar in zeitlicher Hinsicht benachbarten Messintervallen (M100) größer ist als der Zeitraum zumindest eines der Messintervalle (M100), insbesondere wobei in dadurch generierten Messpausen keinerlei Messung stattfindet.

## Claims

1. A method (200) for monitoring a driver of a vehicle by means of a measurement system (1000), the method (200) comprising the steps of:
- Providing at least one measuring system (1000) for measuring pressure and/or humidity, the measuring system (1000) being coupled to or integrated with at least one vehicle element (100A), and the measuring system (1000) comprising at least one sensor (1) for measuring a, preferably only one, stress level of the driver, such that pressure and/or humidity is measured by the sensor (1);
- subsequently forwarding the measured stress level values, i.e. the pressure and/or humidity values, to a processing unit (5) of the measurement system (1000);
- Detecting and recognizing a stress level of the driver based on the stress level values;
- Determining a selected action based on the detection by the measurement system (1000), wherein the action is selected from a list consisting of: establishing a call to a remote support site, transmitting the stress level values to a remote support site, generating an audible alarm and generating a visual alarm, adjusting a volume of speakers in the vehicle, adjusting a seating position of a vehicle seat of the vehicle, and displaying break advisories to the driver, wherein
- within a measurement time interval (M100), the pressure and/or the temperature is measured first and only thereafter the humidity and/or the temperature, wherein within each measurement time interval (M100) the pressure is measured only once and the humidity only once.

2. Method (200) according to claim 1,
**characterized in that**
only pressure and humidity are measured by the sensor (1) to determine a stress level of the driver.

3. Method (200) according to claim 1 or 2,
**characterized in that**
the sensor (1) is installed in a steering wheel and/or a joystick and/or a vehicle seat of a vehicle so that the driver directly touches the steering wheel and/or the joystick and/or the vehicle seat.

4. Method (200) according to at least one of the preceding claims,
**characterized in that**
limit values of pressure and/or humidity are stored in a memory of a CPU (40), wherein the respective pressure and temperature values measured discretely in time or continuously are compared with the values stored in the memory of the CPU (40), wherein if at least one of these values (humidity and pressure) is exceeded, an action is determined by the CPU (40) for execution.

5. Method (200) according to at least one of the preceding claims,
**characterized in that**
factor limit values of pressure and humidity are stored in a memory of the CPU (40), wherein the respective discrete-time or continuously measured pressure and temperature values are compared with the values stored in the memory of the CPU (40), wherein if at least one of these values (humidity and pressure) is exceeded, the CPU (40) determines an action for execution, wherein the factor limit value is defined as a factor of the respective pressure and humidity value, in particular wherein both values are measured at the same time by the sensor (1).

6. Method (200) according to at least one of the preceding claims,
**characterized in that**
within a predetermined measuring time interval (M100), the pressure and the humidity are each measured at different times.

7. Method (200) according to at least one of the preceding claims,
**characterized in that**
a time interval of two measurement intervals (M100) directly adjacent in time is greater than the time period of at least one of the measurement intervals (M100), in particular wherein no measurement takes place in measurement pauses generated thereby.

## Revendications

1. Procédé (200) de surveillance d'un conducteur d'un véhicule au moyen d'un système de mesure (1000), le procédé (200) comprenant les étapes suivantes :
- fournir au moins un système de mesure (1000) pour mesurer la pression et/ou l'humidité, le système de mesure (1000) étant couplé à au moins un élément de véhicule (100A) ou étant intégré à au moins un élément de véhicule, et le système de mesure (1000) comprenant au moins un capteur (1) pour mesurer un, de préférence un seul, niveau de stress du conducteur, de sorte que la pression et/ou l'humidité sont mesurées par le capteur (1) ;
- transmettre ensuite les valeurs de niveau de stress mesurées, c'est-à-dire les valeurs de pression et/ou d'humidité, à une unité de traitement (5) du système de mesure (1000) ;
- Détection et reconnaissance d'un niveau de stress du conducteur basé sur les valeurs de niveau de stress ;
- Déterminer une action sélectionnée sur la base de la détection par le système de mesure (1000), l'action étant sélectionnée dans une liste comprenant : l'établissement d'un appel vers un centre d'assistance distant, la transmission des valeurs de niveau de stress à un centre d'assistance distant, la génération d'une alarme sonore et la génération d'une alarme visuelle, le réglage d'un volume de haut-parleurs dans le véhicule, le réglage d'une position de siège d'un siège de véhicule du véhicule, et l'affichage de recommandations de pause pour le conducteur, où
- à l'intérieur d'un intervalle de temps de mesure (M100), on mesure d'abord la pression et/ou la température et ensuite seulement l'humidité et/ou la température, la pression n'étant mesurée qu'une fois et l'humidité qu'une fois à l'intérieur de chaque intervalle de temps de mesure (M100).

2. procédé (200) selon la revendication 1,
**caractérisé en ce que**
seules la pression et l'humidité sont mesurées par le capteur (1) afin de déterminer un niveau de stress du conducteur.

3. procédé (200) selon la revendication 1 ou 2,
**caractérisé en ce que**
le capteur (1) est monté dans un volant et/ou un joystick et/ou un siège de véhicule d'un véhicule, de sorte que le conducteur touche directement le volant et/ou le joystick et/ou le siège de véhicule.

4. Procédé (200) selon au moins l'une des revendications précédentes,
**caractérisé en ce que**
des valeurs limites de pression et/ou d'humidité sont enregistrées dans une mémoire d'une CPU (40), les valeurs de pression et de température mesurées respectivement de manière discrète dans le temps ou en continu étant comparées aux valeurs enregistrées dans la mémoire de la CPU (40), une action étant déterminée par la CPU (40) pour l'exécution en cas de dépassement d'au moins une de ces valeurs (humidité et pression).

5. Procédé (200) selon au moins l'une des revendications précédentes,
**caractérisé en ce que**
des valeurs limites de facteurs de pression et d'humidité sont enregistrées dans une mémoire de l'unité centrale (40), les valeurs de pression et de température mesurées respectivement de manière discrète dans le temps ou en continu étant comparées aux valeurs enregistrées dans la mémoire de l'unité centrale (40), une action étant déterminée par l'unité centrale (40) pour l'exécution en cas de dépassement d'au moins une de ces valeurs (humidité et pression), la valeur limite de facteur étant définie comme un facteur de la valeur de pression et d'humidité respective, en particulier les deux valeurs étant mesurées au même moment par le capteur (1).

6. Procédé (200) selon au moins l'une des revendications précédentes,
**caractérisé en ce que**
à l'intérieur d'un intervalle de temps de mesure prédéterminé (M100), la pression et l'humidité sont mesurées respectivement à des moments différents.

7. Procédé (200) selon au moins l'une des revendications précédentes,
**caractérisé en ce que**
un intervalle de temps entre deux intervalles de mesure (M100) directement voisins dans le temps est supérieur à la durée d'au moins l'un des intervalles de mesure (M100), en particulier aucune mesure n'étant effectuée pendant les pauses de mesure ainsi générées.
